# EUROPEAN PATENT APPLICATION

(11) **EP 4 306 149 A1**
(43) Date of publication of application: **17.01.2024**
(21) Application number: 22185174.4
(22) Date of filing: 15.07.2022
(51) Int. Cl.: A61M 5/31, A61M 5/32, A61M 5/34

(54) **SYSTEM FOR LONG-TERM STORAGE OF A PHARMACEUTICAL COMPOSITION COMPRISING AN ADAPTER FOR CONNECTING A NEEDLE TO A SYRINGE BARREL**

(71) Applicant: SCHOTT Pharma Schweiz AG, 9000 St. Gallen (CH)
(72) Inventor: Hari, Michael, CH-9503 Braunau (CH); Moser, Raymond, CH-9032 Engelburg (CH); Van Ginneken, Tom, CH-9404 Rorschacherberg (CH); Kern, Patrick, CH-9200 Gossau (CH); Schreiber, Marc, 7576EL Oldenzaal (NL); Torenbeek, Martijn, 7384AR Wilp (NL); Nijsen, Andreas Jacobus Louis, 7521 AT Enschede (NL); Emmens, Philip Ameling, 7552 GG Hengelo (NL)
(74) Representative: Fuchs Patentanwälte Partnerschaft mbB

(57) **Abstract**

Disclosed are an adapter for connecting a needle to a syringe barrel having a threaded Luer Lock connector and a system for long-term storage of a pharmaceutical composition comprising the same.

## Description

The present invention relates to an adapter for connecting a needle to a syringe barrel having a threaded Luer Lock connector and a system for long-term storage of a pharmaceutical composition comprising the same.

### Background

Pharmaceutical or cosmetic compositions are often delivered in a convenient ready-to-use form in prefilled syringes. Typically, such a syringe comprises a syringe barrel filled with the composition, a plunger, a needle hub, and a needle which is typically covered by a needle shield. One method for connecting the needle hub with the syringe barrel uses a standardized Luer Lock connector which comprises a thread and a cone.

Integrity of the closure of the prefilled syringe is of utmost importance in order to avoid spilling and/or contamination of the pharmaceutical or cosmetic composition stored therein. A particularly critical area for leakage is the screwing connector of the Luer Lock. The needle hub which secures the needle against the syringe barrel is screwed on and, hence, can constitute a weak spot of the connection under storage and transport conditions. The connection between the Luer Lock conus and the needle hub is sealed by a form fit between the surfaces of their respective conus surfaces and is may additionally be sealed by an elastomeric sealing member which is compressed by the screwed together parts. During transport of the prefilled syringe, vibrations can loosen the screwing connection leading to an insufficient sealing. In a worst case, the needle can even completely detach from the container.

Further, in situations in which prefilled syringes are stored at reduced temperatures the screwing connection can loosen. The lower the storage temperature or the higher the difference between the filling, storage, and application temperatures, the higher is the risk of a loosening screwing connection and a resulting loss of integrity.

The Luer Lock connector had been specifically designed with a thread connector in addition to the single conus, which is known as the Luer Slip connector, in order to avoid the needle from popping off when higher pressure has to be exerted during injection due to small needle gauges and/or highly viscous injection media. Principally, this design achieves this goal quite well. However, there is still need for improvements in particular for connections of automatic injection devices where a small volume over a prolonged time period is to be dosed and consequently the pressure has to be kept for a long duration. In these cases the screwing connection can start to drift as well and loosen.

Hence, it is an object of the present invention to overcome at least some of the above disadvantages, in particular to improve the sealing integrity of the Luer Lock connection between a syringe barrel and a needle hub.

### Summary of disclosure

### Definitions

Herein:
A "crest" of a thread refers to the prominent top point of the thread.
A "root" of a thread refers to the bottom of the groove between two thread flanks.
A "pitch" of a thread refers to the distance between two neighboring crests along the thread.
A "major outer diameter" of the thread refers to the larger of two extreme diameters delimiting the height of the thread profile. It is the diameter of the thread measured between the opposite crest surfaces and equals to the external diameter of the thread.
A "minor outer diameter" of the thread refers to the smaller of two extreme diameters delimiting the height of the thread profile. It is the diameter of the thread measured between the opposite root surfaces and equals to the diameter of the core of the thread.
A "depth" of the thread refers to the distance between a crest and its neighboring root and is equal to the difference between the major outer diameter and the minor outer diameter of the thread.
A "shape of a cylinder" can be understood in the context of this application as a shape in the form of the mathematic definition of a general cylinder, in particular a general straight cylinder, wherein the definition explicitly includes the special shapes of the prism, in particular the straight prism, and the cuboid. Preferably it is a straight circular cylinder or a straight prism with hexagonal to icositetragonal base areas.
A "form-fit" connection can refer to a joining technique where the joint is effected by geometrical elements which prevent the movement of the components relative to one another. Form-fit connections are created by the interlocking of at least two connection partners. The connecting partners cannot come loose even without or with interrupted force transmission. Under operating load, compressive forces act perpendicular to the surfaces of the connecting partners. Examples are dove-tail joints, tongue and groove joints, keyed joints, or interlocking joints.
A "material-fit" connection can refer to a joining technique where the connecting partners are held together by atomic or molecular forces. At the same time, they are non-detachable connections that can only be separated by destroying the connecting means. Examples are welding, soldering, gluing, or vulcanization.
A "counter thread" refers to a second thread suitable for engaging with a first thread. As such it can be defined by the possibility to screw the two threads into each other. It does not necessarily define the thread as an inner or outer thread. Both versions are equally covered by the definition and are chosen dependent on the design of the first thread.

If a part is said to be "arranged" at some place, this can be meant to refer to the part being positioned there or the other part at this place being provided with the part, i.e. the alternatives of an assembly of two parts and the formation of an integral part are comprised.

### Aspects

In a first aspect the invention relates to an adapter for connecting a needle to a syringe barrel having a thread and a cone, the adapter comprising:
- a first part supporting the needle,
- a second part having a counter thread for a screwing connection with the thread of the Luer Lock connector, and
- at least one deformable locking element arranged within a region of the counter thread and protruding radially beyond a minor outer diameter of the counter thread, such that the at least one deformable locking element is deformed by the thread of the Luer Lock connector when the adapter is screwed to the syringe barrel.

The at least one deformable locking element can have the form of a projection or a protrusion.

"Protruding radially beyond a minor outer diameter of the counter thread" means in this context that the at least one deformable locking element extends in radial direction from the core of the thread, i.e. its minor outer diameter, towards its crests.

Preferably, the deformation of the at least one deformable locking element comprises an axial and/or radial compression.

The "region of the counter thread" in this context refers to essentially the whole surface of the thread, meaning that the deformable locking elements are to be located in a fashion suitable for engaging with the thread of the Luer Lock connector of a syringe barrel.

The adapter can also be referred to as a "needle hub". The needle hub is removable from the syringe barrel and replaceable. It comprises two constructional parts, the first part being a needle base holding the needle and the second part being a gripping base with the thread connector. For assembling the adapter with a syringe it is held at the gripping base and screwed on.

In a second aspect the invention relates to an adapter for connecting a needle to a syringe barrel comprising a Luer Lock connector having a thread and a cone, the adapter comprising:
- a first part supporting the needle,
- a second part having a counter thread for a screwing connection with the thread of the Luer Lock connector, and
- at least one deformable locking element arranged within a region of the counter thread and protruding radially beyond a minor outer diameter of the counter thread, such that the at least one deformable locking element is deformed by the thread of the Luer Lock connector when the adapter is screwed to the syringe barrel.

The Luer Lock connector is preferably a connector according to ISO 80369-7:2021.

In a preferred embodiment, the at least one deformable locking element is elastic and/or comprises, preferably consists of, an elastomeric material. Preferably, the at least one deformable locking element is made of an elastomeric material. Examples of preferred materials are silicone rubber, butyl rubber, and bromobutyl rubber, each optionally containing a mineral filler, in particular a silica.

The inventors have discovered that the integrity of a prefilled syringe can be increased by securing the thread connection between the needle adapter and the Luer Lock connector with at least one additional deformable locking element.

The deformable locking element can be a part of the needle adapter and positioned in a way in or on the thread that it can engage with the thread of Luer Lock connector. When the two parts are screwed together, the deformable locking element can be bent or compressed and a frictional engagement between the two threads counteracts torsional forces created by vibration or temperature induced tension. Depending on the size, shape, and diameter, the deformable locking element engages with the flanks and/or the root of the thread of Luer Lock connector.

Particularly if the deformable locking element is made of an elastomeric material, an enhanced friction can be generated by the material itself and due to the fact that the deformable locking element can adopt the shape of the space between the threads. Such an embodiment can, for example, advantageously be provided by means of two component injection molding the second part and the deformable locking element using a thermoplastic elastomer (TPE).

In a further preferred embodiment, the at least one deformable locking element can be at least one of a cam, a bulge, a projecting rim, a projecting lip, or a bead; and/or the adapter comprises a plurality of deformable locking elements. The plurality of deformable locking elements can be two, three, four, five, six, or more deformable locking elements. Preferred are two, three, or four deformable locking elements, particularly preferred two or three. It can, for example, be not more than ten deformable locking elements since dimensioning and positioning of too many deformable locking elements can get impracticable or uneconomical. Consequently, embodiments can preferably have two to ten deformable locking elements, more preferably two to six deformable locking elements, two to five deformable locking elements, or two to four deformable locking elements.

Preferably, the at least one deformable locking element can also protrude radially beyond a major outer diameter of the counter thread. If the deformable locking element protrudes beyond the thread, it is possible to create more compression of the deformable locking element when the threads are screwed together. Further, the tip of the deformable locking element can easier reach the root of the thread of Luer Lock connector and engage with it since generally the crown of these threads does not extend to the root of the mating thread. Hence, if the deformable locking element does not extend beyond the crown, i.e. the major outer diameter, in its initial state, it can only engage with the root if it gets compressed enough by the mating thread to extend beyond the thread. Otherwise it will only engage with the flanks of the thread.

In a third aspect of the invention, the adapter can comprise a third part including the at least one deformable locking element and including one or more inner surface(s) for contacting the cone of the Luer Lock connector, wherein the second part has at least one opening formed within the region of the counter thread; and the third part is arranged at least partially inside the second part and the at least one deformable locking element engages with and extends through the at least one opening. Such an embodiment provides the advantage that the third part can be made of another material than the second part. In particular, it can be made of a material which provides tailored properties for the locking function and a good sealing against the cone of the Luer Lock connector while the second part is optimized for stability of the adapter.

The at least one opening formed within the region of the counter thread can extend in radial direction through the complete wall thickness of the second part in the region of the thread, most preferably including the thread.

In preferred embodiments, the third part comprises, preferably consists of, a polymer, preferably an elastomer, more preferably a thermoplastic elastomer. Examples of preferred materials are silicone rubber, butyl rubber, and bromobutyl rubber, each optionally containing a mineral filler, in particular a silica. Preferably, the material is the same material as that of the at least one deformable locking element.

Preferably, the at least one deformable locking element is arranged substantially perpendicular to a central axis of the counter thread of the second part when assembled. This is advantageous because the compression of the thread connection then can exert forces in axial direction with the root of the thread acting on the full length of the deformable locking element in addition to the radial forces effected by the flanks of the thread. If the orientation is not perpendicular, a bending momentum is introduced which reduces the compression.

In preferred embodiments, the first part and the second part form a form-fit or material-fit integral part. In these embodiments, the two parts are secured to each other by means of a locking mechanism or a material bond in order to prevent an unintentional separation of the parts which would result in a full loss of the integrity of a prefilled syringe with the adapter according to the invention and a spilling of the contents.

In further preferred embodiments, the first part and the second part are irreversibly connected, preferably by a click or snapping mechanism. The connection can be rotatable around the central axis of the needle, particularly in the case of a click or snapping mechanism. An irreversible connection is to be preferred due to the fact that this way not only the above mentioned unintentional separation is prevented but also any tempering with the adapter parts is prevented. A user of the adapter generally has no reason to disassemble it since the needle will be replaced together with the whole adapter in order to maintain sterility of the assembly. A click or snapping mechanism can, for example, be achieved by notches, latches and/or undercuts.

In a preferred variant of this embodiment, wherein the first part and the second part are irreversibly connected by a click mechanism, the setting force to reach the click point of the click mechanism is 10 N to 300 N, preferably 20 N to 200 N, more preferably 80 N to 150 N. In embodiments, the setting force can be at least 10 N, preferably at least 20 N or at least 80 N. The setting force can be at most 300 N, preferably at most 200 N or at most 150 N.

Preferably, the at least one deformable locking element has an elliptic or rectangular cross-sectional shape, preferably a circular or a square cross-sectional shape. These cross-sectional shapes have proven themselves as particularly suitable for adjusting the locking effect and assuring good contact with the thread. An adjustment of the locking effect is required for tailoring the holding forces and the screwing torque as the adapter is expected not only to be safely held in place assuring the integrity but also to still be conveniently screwable by the user.

Further, in preferred embodiments a distal end of the at least one deformable locking element has in a not deformed state, preferably uncompressed state, a shape of a cylinder, particularly a prism or a cuboid, preferably a cube, of a frustum of a cone, of a frustum of a pyramid, and/or of a hemi-ellipsoid, particularly a hemisphere. These forms of the tip of the deformable locking element are advantageous for the contact with the root of the thread and also the adjustment of the locking effect. The "and" alternative of the list is particularly referring to the combination of one of the first three shapes with the fourth shape resulting in rounded tips. However, other combinations are also useful and contemplated alike, for example a cylinder with a frustum of a cone which can serve for a better match with the shape of the root of the thread.

In some preferred embodiments, a ratio between a diameter or width of the at least one deformable locking element extending in the direction of the central axis of the counter thread and a pitch of the counter thread of the second part is from 0.3 to 0.5 [mm/mm]. This ratio characterizes the amount by which the deformable locking element laterally extends beyond the thread, in other words, how much broader than the thread is the deformable locking element. Since the thread matches that of the Luer Lock thread, this is at the same indicative for the amount of compression the deformable locking element will receive from the Luer Lock thread. Configurations within the ratio range of 0.3 to 0.5 [mm/mm] have been found to provide optimal contact and compression forces for a secure fixation of the adapter at a convenient required screw on torque. The ratio can preferably be at least 0.3 [mm/mm], at least 0.31 [mm/mm], at least 0.32 [mm/mm], at least 0.33 [mm/mm], at least 0.34 [mm/mm], or at least 0.35 [mm/mm]. The ratio can preferably be at most 0.5 [mm/mm], at most 0.49 [mm/mm], at most 0.48 [mm/mm], at most 0.47 [mm/mm], at most 0.46 [mm/mm], or at most 0.45 [mm/mm]. The ratio can preferably be within the ratio range of 0.3 to 0.5 [mm/mm], within the ratio range of 0.31 to 0.49 [mm/mm], within the ratio range of 0.32 to 0.48 [mm/mm], within the ratio range of 0.33 to 0.47 [mm/mm], within the ratio range of 0.34 to 0.46 [mm/mm], or within the ratio range of 0.35 to 0.45 [mm/mm].

Another ratio which can be observed for the same purpose is the ratio between a diameter or width of the at least one deformable locking element extending in the direction of the central axis of the counter thread and a crest of the counter thread of the second part. However, this crest ratio does not comprise information about the size of the root of the thread and is, hence, less informative than the before mentioned pitch ratio. Preferably, the ratio is from 1 to 8 [mm/mm], more preferably from 1.1 to 7.7 [mm/mm].

In preferred embodiments, the at least one deformable locking element extends beyond the major outer diameter of the counter thread of the second part by 0.0 mm - 1.5 mm, preferably by 0.1 mm - 0.5 mm. Preferably, it can extend it by at least 0.0 mm, at least 0.025 mm, at least 0.05 mm, at least 0.075 mm, or at least 0.1 mm. Preferably, it can extend it by at most 1.5 mm, at most 1.25 mm, at most 1.0 mm, at most 0.75 mm, or at most 0.5 mm. Preferably, it can extend it by 0.0 mm - 1.5 mm, by 0.025 mm - 1.25 mm, by 0.05 mm - 1.0 mm, by 0.075 mm - 0.75 mm, or by 0.1 mm - 0.5 mm. This range of length by which the deformable locking elements exceed the height of the counter thread is indicative for the axial compression exerted by the thread flanks and particularly the root of the Luer Lock thread and has been found to provide optimal contact and compression forces for a secure fixation of the adapter at a convenient required screw on torque.

In preferred embodiments, at least the at least one deformable locking element, preferably the whole third part, has a Shore A hardness, measured according to ASTM D2240:2021, 10 seconds, of 20 to 80, preferably 30 to 70, more preferably 45 to 65, more preferably 50 to 60. The Shore A hardness can preferably be at least 20, at least 30, at least 45, or at least 50. The Shore A hardness can preferably be at most 80, at most 70, at most 65, or at most 60.

In preferred embodiments, a Young's modulus of the at least one deformable locking element and/or the third part is from 0.1 MPa to 5 MPa, preferably from 1 MPa to 4 MPa, more preferably from 1.5 MPa to 3 MPa, determined according to ISO 527-1/-2:2019. The Young's modulus can preferably be at least 0.1 MPa, at least 0.5 MPa, at least 1 MPa, at least 1.25 MPa or at least 1.5 MPa. The Young's modulus can preferably be at most 5 MPa, at most 4.5 MPa, at most 4 MPa, at most 3.5 MPa or at most 3 MPa. The Young's modulus can preferably be from 0.1 MPa to 5 MPa, from 0.5 MPa to 4.5 MPa, from 1 MPa to 4 MPa, from 1.25 MPa to 3.5 MPa, or from 1.5 MPa to 3 MPa.

In embodiments, the adapter has a frictional torque resistance of 0.9 Ncm - 4 Ncm, preferably 1 Ncm - 3.5 Ncm, when assembled to a Luer Lock connector with the cone removed and measured according to ISO 11040-4:2015, Annex G.4. The frictional torque resistance is preferably at least 0.9 Ncm, at least 0.92 Ncm, at least 0.94 Ncm, at least 0.96 Ncm, at least 0.98 Ncm, or at least 1 Ncm. The frictional torque resistance is preferably at most 4 Ncm, at most 3.9 Ncm, at most 3.8 Ncm, at most 3.7 Ncm, at most 3.6 Ncm, or at most 3.5 Ncm. The frictional torque resistance is preferably 0.9 Ncm - 4 Ncm, 0.92 Ncm - 3.9 Ncm, 0.94 Ncm - 3.8 Ncm, 0.96 Ncm - 3.7 Ncm, 0.98 Ncm - 3.6 Ncm, or 1 Ncm - 3.5 Ncm. In order to be able to measure the frictional torque resistance imparted by the at least one deformable locking element, the cone of the Luer Lock connector of the test syringe has to be removed before measuring. The remainder of the measurement conditions can then be used as in the ISO norm. The inventors have found that a frictional torque resistance within this range provides a secure fixation and a comfortable operation of the adapter.

In preferred embodiments, the at least one opening for the at least one deformable locking element in the region of the counter thread of the second part are arranged at a position along the length of the region of the counter thread when viewed from the distal end to the proximal end of the second part within 20 % - 100 % of the length, preferably within 75 % - 100 %. The position is preferably at at least 20 % of the length, at least 30 % of the length, at least 40 % of the length, at least 50 % of the length, at least 60 % of the length, at least 70 % of the length, or at least 75 % of the length. The position is preferably within 20 % - 100 % of the length, within 30 % - 100 % of the length, within 40 % - 100 % of the length, within 50 % - 100 % of the length, within 60 % - 100 % of the length, within 70 % - 100 % of the length, or within 75 % - 100 % of the length. It has been found that a position of the at least one deformable locking element closer to the end of the counter thread of the second part, i.e. closer to the syringe body in its assembled position, provides a larger impact on the torque and a more even screwing experience for the user.

In preferred embodiments, the at least one deformable locking element has in a not deformed state, preferably uncompressed state, a surface area for contacting the thread of the Luer Lock connector of 1.5 mm² - 4.0 mm², preferably 2.5 mm² - 3.5 mm². The surface area is preferably at least 1.5 mm², at least 1.75 mm², at least 2.0 mm², at least 2.25 mm², or at least 2.5 mm². The surface area is preferably at most 4.0 mm², at most 3.9 mm², at most 3.8 mm², at most 3.7 mm², at most 3.6 mm², or at most 3.5 mm². The surface area is preferably 1.5 mm² - 4.0 mm², 1.75 mm² - 3.9 mm², 2.0 mm² - 3.8 mm², 2.25 mm² - 3.7 mm², 2.5 mm² - 3.6 mm², or 2.5 mm² - 3.5 mm². Within this range, the contact surface of the deformable locking element ideally suited for the locking function. The surface is large enough to provide sufficient friction on the thread but still small enough to avoid the occurrence of too high frictional torque resistance and too much compression by the thread.

Most preferably, the material of the first part and/or the second part comprises, preferably consists of, a polymer, preferably selected from the group comprising polyamide (PA), polypropylene (PE), polycarbonate (PC), polyvinylidene fluoride (PVDF), cyclic olefin copolymers (COC), cyclic olefin polymers (COP), and polyethylene terephthalate (PET).

In preferred embodiments, the needle and the first part are an integral part, wherein preferably the needle is overmolded by injection molding with the first part. The integration of the needle into the first part can be achieved by different mechanisms. The needle can be fixed with an adhesive into the first part or by means of a press-fit connection. The preferred way is the fixation by means of overmolding the needle in an injection molding process since this saves a further assembly step and achieves a superb fixation with optimal sealing.

In embodiments, the adapter is configured such that a fluid which is injected from the syringe barrel to the needle contacts after leaving the cone only the first part and/or the third part of the adapter before entering the needle. Such a design variant has the advantage that not all parts of the adapter have to be made of expensive medical grade polymers. In general, the first part will be of smaller size than the second part since the latter has to be large enough for convenient handling by a user while the former needs only a size sufficient for fixing the needle in the second part. Also the third part is of smaller size since it will be positioned within the second part. Hence, if the design of the adapter is such that the fluid of the syringe will not come into contact with the second part on its way into the needle, only the first and/or third part will have to be made of a medical grade polymer. Even if the size of the three parts is about equal, costs can still be cut down because only one or two of them will require the expensive material. For example, the first part can be made of medical grade COC while the second part is made of standard grade PA or PC if the fluid enters directly from the cone of the Luer Lock connector into the first part.

In further embodiments, the adapter is configured such that a ratio between a hardness of the at least one deformable locking element determined as Shore A, 3 s according to DIN ISO 7619-1:2012-02 and a hardness of the thread of the Luer Lock of the container determined as Shore D, 3 s according to DIN ISO 7619-1:2012-02 is from 1:1.1 to 1:2.0, preferably from 1:1.2 to 1:1.6. The inventors have found that the effect of the deformable locking element can be improved if its hardness is chosen within a certain ratio to the hardness of the thread of the Luer Lock of the container on which it will abut.

In a fourth aspect, the invention relates to a system for long-term storage of a pharmaceutical composition, comprising:
- a syringe barrel, comprising:
   + a front end,
   + a Luer Lock connector comprising a thread and a cone, preferably according to ISO 80369-7:2021, provided at the front end, and
   + an opposite back end;
- a plunger inserted into the back end;
- an adapter according to the invention connected to the Luer Lock connector at the front end; and
- optionally a needle shield covering the needle.

Such a system comprising an adapter according to the invention improves the integrity of a prefilled syringe during long-term storage, particularly at reduced temperatures, and transport. The deformable locking elements will secure the adapter on the Luer Lock connector of the syringe and prevent it from loosening or coming off.

In preferred embodiments, the syringe barrel comprises, preferably consists of, a glass, preferably a borosilicate or aluminosilicate glass, or a polymer, preferably polypropylene (PE), polypropylene (PP), cyclic olefin copolymers (COC), or cyclic olefin polymers (COP).

In preferred embodiments, the syringe barrel is filled with a solid or a liquid composition, preferably a pharmaceutical composition.

### Brief description of the drawings

- Figure 1: shows a lateral view of a front end of a syringe barrel having a threaded Luer Lock connector and a two-part embodiment of an adapter according to the invention for connecting a needle to it.
- Figure 2: shows a cross-sectional view of a front end of a syringe barrel having a threaded Luer Lock connector and a three-part embodiment of an adapter according to the invention for connecting a needle to it.
- Figure 3: shows an enlarged perspective view of the threaded Luer Lock connector and an inserted third part.

### Detailed description

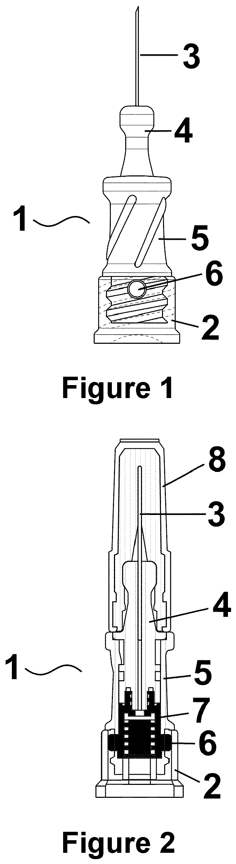

A first example of an adapter 1 according to the invention for connecting a needle 3 to a syringe barrel having a threaded Luer Lock connector 2 is shown in Figure 1. It is an example of a two-part design of the adapter 1 with a deformable locking element 6 in the form of a hemispherical cam made of an elastomer which has been glued to the root of the counter thread of the second part 5. A part of the counter thread has been removed for this purpose. The figure shows only the front end of a syringe barrel with the Luer Lock connector 2. Not shown are the rest of the syringe barrel and the plunger which complete the figure to a system according to the invention.

The first part 4 is made of a cyclic olefin copolymer (COC) by injection molding wherein the needle 3 has been overmolded to fix it in the first part 4. The first part 4 and the second part 5, which has been made of polycarbonate (PC), have been glued together to form an integral part. The second part 5 is then screwed in the thread of the Luer Lock connector 2 where it is secured by the deformable locking element 6.

A second example of an adapter 1 according to the invention is shown in **Figure 2****.** This time the adapter 1 is comprising three parts. While from the outside looking almost identical to the example of Figure 1, the cross-sectional view reveals the differences. Again, the figure does not show the complete syringe barrel and the plunger of the system but it does show an optional needle shield 8 covering the first part 4 and the needle 3.

As in the example of Figure 1, the first part 4 is made of a cyclic olefin copolymer (COC) by injection molding wherein the needle 3 has been overmolded to fix it in the first part 4. However, the section of the first part 4 which rests inside the second part 5 is shaped with a projecting ring (see the protrusions on the lower cylindrical section in Figure 2). The second part 5 is also made of a cyclic olefin copolymer (COC) and has on its inner circumference a corresponding undercut for interlocking with the projecting ring when the first part 4 and the second part 5 are pushed together for assembly. This irreversible connection can be designed to be rotatable or non-rotatable by adjusting the distance between the projecting ring and the flange resting on the upper end of the second part 5 relative to the distance between the corresponding undercut and the rim where the flange abuts.

The third part 7 is made of a bromobutyl rubber having a hardness of 50 Shore A (measured according to ASTM D2240:2021, 10 seconds) and positioned inside of the second part 5. As opposed to the first example, the third part 7 has two projecting cams arranged opposite to each other with a hemispherical tip. These projecting cams extend through corresponding bores in the second part 5 to form a deformable locking element 6. The bores are positioned at a position of 75 % along the length of the region of the counter thread when viewed from the distal end to the proximal end of the second part 5.The deformable locking elements 6 extend beyond the major outer diameter of the counter thread of the second part 5 by 0.6 mm. The surface area for contacting the thread of the Luer Lock connector 2 in an uncompressed state has been 3.2 mm². When viewed from outside, the deformable locking elements 6 look like in the first example in Figure 1 as the only difference is that the gap in the counter thread of the second part 5 does not stop at the root of the thread but continues in the bore through the wall of the second part 5. The third part 7 abuts with its inner surface on the surface of the cone of the Luer Lock connector 2 as well as the lower end of the first part 4 providing additional sealing of the system.

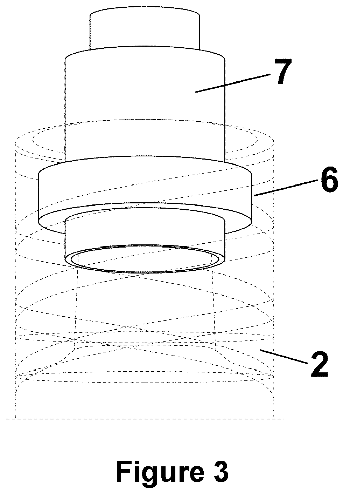

An enlarged perspective view of the threaded Luer Lock connector 2 of the second example shown in Figure 2 and an inserted third part 7 alone is shown in **Figure 3** for better visibility. It can clearly be seen in this figure how the deformable locking elements 6 move in the thread of the Luer Lock connector 2 and effect a frictional force for locking the screwing connection.

With the adapter as shown in Figure 2, torque measurements according to ISO 11040-4:2015, Annex G.4 have been conducted, wherein the cone of the Luer Lock connector 2 has been removed in order to be able to measure the effect of the deformable locking elements 6 without the additional contact surfaces of the third part 7 with the cone. Eleven samples have been measured for calculating the average. For comparison, each of the adapters 1 with the deformable locking elements 6 cut from the third part 7 has been tested.

| **Frictional torque resistance** | **Example 2** | **Example 2 with locking elements cut off** |
|---|---|---|
| Minimum value | 1.03 Ncm | 0.85 Ncm |
| Maximum value | 2.94 Ncm | 1.57 Ncm |
| X | 1.49 Ncm | 1.15 Ncm |
| Median | 1.37 Ncm | 1.20 Ncm |
| Standard deviation | 0.54 Ncm | 0.21 Ncm |

As can be seen in the table, the deformable locking elements 6 provide an increase of about 30 % in the frictional torque resistance when screwing the adapter 1 of example 2 in a Luer Lock connector 2.

### Reference number list

- 1: Adapter
- 2: Luer Lock connector
- 3: Needle
- 4: First part
- 5: Second Part
- 6: Deformable locking element
- 7: Third part
- 8: Needle shield

## Claims

1. Adapter (1) for connecting a needle (3) to a syringe barrel comprising a Luer Lock connector (2) having a thread and a cone, the adapter (1) comprising:
- a first part (4) supporting the needle (3),
- a second part (5) having a counter thread for a screwing connection with the thread of the Luer Lock connector (2), and
- at least one deformable locking element (6) arranged within a region of the counter thread and protruding radially beyond a minor outer diameter of the counter thread, such that the at least one deformable locking element (6) is deformed by the thread of the Luer Lock connector (2) when the adapter (1) is screwed to the syringe barrel.

2. Adapter according to claim 1, wherein
- the at least one deformable locking element (6) is elastic and/or comprises, preferably consists of, an elastomeric material; and/or
- the at least one deformable locking element (6) is at least one of a cam, a bulge, a projecting rim, a projecting lip, or a bead; and/or
- the adapter (1) comprises a plurality of deformable locking elements (6).

3. Adapter (1) according to one of the preceding claims, wherein the at least one deformable locking element (6) is also protruding radially beyond a major outer diameter of the counter thread.

4. Adapter (1) according to one of the preceding claims, comprising
- a third part (7) including the at least one deformable locking element (6) and including one or more inner surface(s) for contacting the cone of the Luer Lock connector (2),
wherein
- the second part (5) has at least one opening formed within the region of the counter thread; and
- the third part (7) is arranged at least partially inside the second part (5) and the at least one deformable locking element (6) engages with and extends through the at least one opening.

5. Adapter (1) according to claim 4, wherein the third part (7) comprises, preferably consists of, a polymer, preferably an elastomer, more preferably a thermoplastic elastomer.

6. Adapter (1) according to one of the preceding claims, wherein the at least one deformable locking element (6) is arranged substantially perpendicular to a central axis of the counter thread of the second part (5) when assembled.

7. Adapter (1) according to one of the preceding claims, wherein the first part (4) and the second part (5) form a form-fit or material-fit integral part and/or are irreversibly connected, preferably by a click or snapping mechanism.

8. Adapter (1) according to claim 7, wherein the first part (4) and the second part (5) are irreversibly connected by a click mechanism and the setting force to reach the click point of the click mechanism is 10 N to 300 N, preferably 20 N to 200 N, more preferably 80 N to 150 N.

9. Adapter (1) according to one of the preceding claims, wherein the at least one deformable locking element (6)
- has an elliptic or rectangular cross-sectional shape, preferably a circular or a square cross-sectional shape; and/or
- extends beyond the major outer diameter of the counter thread of the second part (5) by 0.0 mm - 1.5 mm, preferably by 0.1 mm - 0.5 mm; and/or
- has a Shore A hardness, measured according to ASTM D2240:2021, 10 seconds, of 20 to 80, preferably 30 to 70, more preferably 45 to 65, more preferably 50 to 60; and/or
- has in a not deformed state, preferably uncompressed state, a surface area for contacting the thread of the Luer Lock connector (2) of 1.5 mm² - 4.0 mm², preferably 2.5 mm² - 3.5 mm²; and/or
- has a Young's modulus from 0.1 MPa to 5 MPa, preferably from 1 MPa to 4 MPa, more preferably from 1.5 MPa to 3 MPa, determined according to ISO 527-1/-2:2019.

10. Adapter (1) according to one of the preceding claims, wherein a ratio between a diameter or width of the at least one deformable locking element (6) extending in the direction of the central axis of the counter thread and a pitch of the counter thread of the second part (5) is from 0.3 to 0.5 [mm/mm].

11. Adapter (1) according to one of the preceding claims, wherein the adapter (1) has a frictional torque resistance of 0.9 Ncm - 4 Ncm, preferably 1 Ncm - 3.5 Ncm, when assembled to a Luer Lock connector (2) with the cone removed and measured according to ISO 11040-4:2015, Annex G.4.

12. Adapter (1) according to one of the preceding claims, wherein the at least one opening for the at least one deformable locking element (6) in the region of the counter thread of the second part (5) are arranged at a position along the length of the region of the counter thread when viewed from the distal end to the proximal end of the second part (5) within 20 % - 100 % of the length, preferably within 75 % - 100 %.

13. Adapter (1) according to one of the preceding claims, wherein a distal end of the at least one deformable locking element (6) has in a not deformed state, preferably uncompressed state, a shape
- of a cylinder, particularly a prism or a cuboid, preferably a cube,
- of a frustum of a cone,
- of a frustum of a pyramid, and/or
- of a hemi-ellipsoid, particularly a hemisphere.

14. System for long-term storage of a pharmaceutical composition, comprising:
- a syringe barrel, comprising:
+ a front end,
+ a Luer Lock connector (2) comprising a thread and a cone, preferably according to ISO 80369-7:2021, provided at the front end, and
+ an opposite back end;
- a plunger inserted into the back end;
- an adapter (1) according to one of the preceding claims connected to the Luer Lock connector (2) at the front end; and
- optionally a needle shield (8) covering the needle (3).

15. System according to claim 14, wherein the syringe barrel
- comprises, preferably consists of, a glass, preferably a borosilicate or aluminosilicate glass, or a polymer, preferably polypropylene, polypropylene, cyclic olefin copolymers, or cyclic olefin polymers; and/or
- is filled with a solid or a liquid composition, preferably a pharmaceutical composition.
